# EUROPEAN PATENT APPLICATION

(11) **EP 3 241 887 A1**
(43) Date of publication of application: **08.11.2017**
(21) Application number: 16182265.5
(22) Date of filing: 01.08.2016
(51) Int. Cl.: C11D 1/835, A61K 8/00, C11D 11/00, C11D 17/00

(54) **COMPOSITION COMPRISING ALCOHOL ETHOXYLATE AND GLUCAMIDE**

(71) Applicant: Clariant International Ltd, 4132 Muttenz (CH)
(72) Inventor: SIENKIEWICZ, Aleksandra, 64283 Darmstadt (DE)
(74) Representative: Paczkowski, Marcus

(57) **Abstract**

Compositions comprising
a) 5 to 50 % by weight of one or more specific alcohol ethoxylates,
b) 15 to 50 % by weight of one or more specific N-methyl glucamides and
c) 25 to 45 % by weight of water
are described.

The compositions may be present, at a temperature of 20 °C, in the form of a gel or in the form of a paste, and furthermore e.g. in the form of a monodose product, and are suitable for cleaning, scenting or disinfection purposes, preferably in home or personal care applications.

## Description

This invention relates to compositions comprising specific alcohol ethoxylates, specific N-methyl glucamides and water, to the use of these inventive compositions for cleaning, scenting or disinfection purposes and to a process for the preparation of these inventive compositions.

Detergents and cleaning agents are commonly present as spray-dried or granulated products or as liquid forms, but following the new tendencies of the growing premium segment and the desire of the consumer for other possibilities of a comfortable dosage, the search for new formats for detergent and cleaning agents has significantly increased.

Nowadays, monodose products are quite popular in home care applications such as in sanitary applications, e.g. for in-bowl toilet cleaners, in automatic dishwashing or laundry applications. The monodose products frequently are present as tablets or pouches comprising powders, liquids or gels. However, the chemical composition of these monodose products, in particular of the gels, is often very complex and is in need of substances such as thickeners. This in turn enhances the complexity of their production and also the costs associated therewith.

It is an object of the present invention to provide new compositions which may be present, at a temperature of 20 °C, in the form of a gel or in the form of a paste, and furthermore e.g. in a monodose format, and are suitable for cleaning, scenting or disinfection purposes, preferably in home or personal care applications.

Surprisingly it has now be found that this object can be solved with compositions comprising
a) 5 to 50 % by weight and preferably 20 to 50 % by weight of one or more alcohol ethoxylates of the formula (I)

   R¹-O-(CH₂CH₂O)ₙ-H (I)

   wherein
   - R¹: is a linear or branched, preferably a linear, saturated alkyl group comprising 12 to 22, preferably 12 to 20 and more preferably 16 to 18, carbon atoms or a linear or branched, preferably a linear, mono- or polyunsaturated alkenyl group comprising 12 to 22, preferably 12 to 20 and more preferably 16 to 18, carbon atoms,
   whereby the alcohol R¹-OH underlying the respective alcohol ethoxylate of the formula (I) possesses a iodine number of from 30 to 135 g(J₂)/100 g alcohol R¹-OH and preferably of from 36 to 120 g(J₂)/100g alcohol R¹-OH, and
   - n: on a molar average, is a number of from 4 to 50 and preferably of from 5 to 25, and
b) 15 to 50 % by weight and preferably 17 to 44 % by weight of one or more N-methyl glucamides of the formula (II) 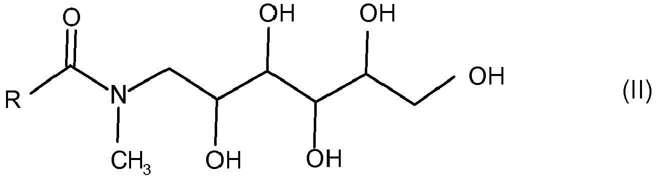 wherein
   - R: is a linear or branched, preferably a linear, saturated alkyl group comprising 7 to 21 and preferably 7 to 17 carbon atoms or a linear or branched, preferably a linear, mono- or polyunsaturated alkenyl group comprising 7 to 21 and preferably 7 to 17 carbon atoms,
   whereby in at least 25.0 wt.-%, preferably in at least 50.0 wt.-%, more preferably in at least 65.0 wt.-% and even more preferably in at least 80.0 wt.-% of the fatty acids R-COOH underlying the respective
   N-methyl glucamides of the formula (II) R is a linear or branched, preferably a linear, saturated alkyl group comprising 11 to 21 and preferably 11 to 17 carbon atoms or a linear or branched, preferably a linear, mono- or polyunsaturated alkenyl group comprising 11 to 21 and preferably 11 to 17 carbon atoms, and
c) 25 to 45 % by weight and preferably 30 to 45 % by weight of water,
the amounts of the of the one or more alcohol ethoxylates of the formula (I) of component a), of the one or more N-methyl glucamides of the formula (II) of component b) and of water of component c) being based on the total weight of the composition.

Therefore, a subject matter of the present invention is compositions comprising
a) 5 to 50 % by weight and preferably 20 to 50 % by weight of one or more alcohol ethoxylates of the formula (I)

   R¹-O-(CH₂CH₂O)ₙ-H (I)

   wherein
   - R¹: is a linear or branched, preferably a linear, saturated alkyl group comprising 12 to 22, preferably 12 to 20 and more preferably 16 to 18, carbon atoms or a linear or branched, preferably a linear, mono- or polyunsaturated alkenyl group comprising 12 to 22, preferably 12 to 20 and more preferably 16 to 18, carbon atoms,
   whereby the alcohol R¹-OH underlying the respective alcohol ethoxylate of the formula (I) possesses a iodine number of from 30 to 135 g(J₂)/100 g alcohol R¹-OH and preferably of from 36 to 120 g(J₂)/100g alcohol R¹-OH, and
   - n: on a molar average, is a number of from 4 to 50 and preferably of from 5 to 25, and
b) 15 to 50 % by weight and preferably 17 to 44 % by weight of one or more N-methyl glucamides of the formula (II) 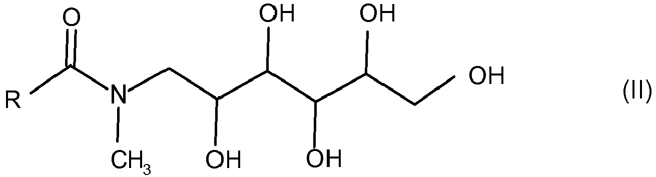 wherein
   - R: is a linear or branched, preferably a linear, saturated alkyl group comprising 7 to 21 and preferably 7 to 17 carbon atoms or a linear or branched, preferably a linear, mono- or polyunsaturated alkenyl group comprising 7 to 21, preferably 7 to 17 carbon atoms,
   whereby in at least 25.0 wt.-%, preferably in at least 50.0 wt.-%, more preferably in at least 65.0 wt.-% and even more preferably in at least 80.0 wt.-% of the fatty acids R-COOH underlying the respective N-methyl glucamides of the formula (II) R is a linear or branched, preferably a linear, saturated alkyl group comprising 11 to 21 and preferably 11 to 17 carbon atoms or a linear or branched, preferably a linear, mono- or polyunsaturated alkenyl group comprising 11 to 21 and preferably 11 to 17 carbon atoms, and
c) 25 to 45 % by weight and preferably 30 to 45 % by weight of water,
the amounts of the of the one or more alcohol ethoxylates of the formula (I) of component a), of the one or more N-methyl glucamides of the formula (II) of component b) and of water of component c) being based on the total weight of the composition.

The inventive compositions may comprise different alcohol ethoxylate molecules according to the formula (I) of component a) with different residues R¹ and/or with different ethoxylation degrees. Furthermore, the inventive compositions may comprise different N-methyl glucamide molecules according to the formula (II) with different residues R.

The above-mentioned amounts stating that in at least 25.0 wt.-%, preferably in at least 50.0 wt.-%, more preferably in at least 65.0 wt.-% and even more preferably in at least 80.0 wt.-% of the fatty acids R-COOH underlying the respective N-methyl glucamides of the formula (II) R is a linear or branched, preferably a linear, saturated alkyl group comprising 11 to 21 and preferably 11 to 17 carbon atoms or a linear or branched, preferably a linear, mono- or polyunsaturated alkenyl group comprising 11 to 21 and preferably 11 to 17 carbon atoms, are based on the total amount of fatty acids R-COOH underlying the one or more N-methyl glucamides of the formula (II) of component b) of the inventive compositions.

The alcohol ethoxylates of the formula (I) and the N-methyl glucamides of the formula (II) may be prepared by methods well-known to the person skilled in the art or are commercially available.

In WO 2010/022975 A2 a gel surfactant composition is described suitable for hard surface cleaning, washing clothes and dishes, and which can be employed for household, institutional and/or industrial applications, composed by water and a) nonionic surfactants in the range of 1 to 50 %, b) cationic surfactant or association of cationic surfactants in the range of 20 to 50 % and c) optionally amphoteric surfactants.

WO 2012/113671 A1 discloses an adhesive composition in gel or paste form for cleaning and/or fragrancing of a WC, which is applied to the inside of the WC ceramic and is rinsed off only after several flush operations, comprises at least one surfactant from the group consisting of alkyl polyglycosides, amphoacetates, amphodiacetates, betaines, fatty alcohol ether carboxylic acids, fatty acid sarcosinates, cocamidopropylamine oxide, aminopropionates, and biosurfactants, perfume and water, and also an ester of polyisobutene succinic acid as an adhesion promoter, and is free of further adhesion promoters and surfactants.

WO 02/26925 A1 describes a sanitary product for cleaning and/or disinfecting and/or releasing fragrances. Said product can be applied directly to the sanitary object, adhering thereto, and can only be removed after many flushing processes. The product contains water; anionic and/or non-ionic and/or amphoteric surfactants; an adhesive agent from the group consisting of polyalkoxyalkanes, alginates, diurethanes, gelatines, pectins, oleyamines, alkyldimethylaminoxides, stearates, sulfonates, sulfates and carbonates; at least one aliphatic di-, oligo-, or polyhydroxy compound or the ether of the same; and optionally other standard consistuents such as thickeners, dyes, preservatives and fragrances. The viscosity of the product is measured as at least 15,000 mPa · s, by means of a Haake viscometer (cone/plate system, sensor PK 5 1°), at a shear gradient of 25 s⁻¹ and at room temperature.

WO 99/66017 discloses a sanitary agent for cleaning and/or disinfecting and/or releasing an odorant, said agent comprising an adhesion promoter, water, anionic and/or non-ionic and/or amphoteric surface active agents and, optionally, further common constituents such as odorants, thickeners, colorants and preservatives. The adhesion promoter is selected from the group of very long or long-chained organic molecules which are hydrophilic at least in part. The hydrophilic part of the adhesion promoter interacts at least in part with the water molecules in the presence of water and becomes "sticky" so that the agent can be applied directly on the sanitary object in the presence of small amounts of water and can adhere thereto. The viscosity of the agent is of at least 15,000 mPa · s and the product can only be washed away completely after numerous rinsings. WO 99/66017 also relates to a method for applying a sanitary agent, wherein the agent is directly applied on the surface of the sanitary object to be cleaned and adheres to said surface.

WO 99/24549 describes a detergent tablet that comprises: A) a compressed solid body portion having at least one mold in the compressed body portion; and B) a non-compressed, gelatinous portion integrally mounted in the mold of the compressed body portion, wherein the gelatinous portion comprising a thickening system and at least one detergent active. The thickening system preferably includes a non-aqueous diluent and a gelling agent and the detergent active is preferably selected from the group consisting of enzymes, surfactants, effervescing agents, bleaching agents, silver care agents, builders, and mixtures thereof.

The present invention in particular concerns a gel or paste material obtained by the association of alcohol ethoxylate of the formula (I), N-methyl glucamide of the formula (II) and water in determined amounts and without the necessity of any thickener or hardener system addition.

In the inventive compositions the combination of alcohol ethoxylate of the formula (I) with N-methyl glucamide of the formula (II) preferably results in homogenous gels with surprisingly high viscosity.

The gel aspect is generated by the association of alcohol ethoxylate of the formula (I), N-methyl glucamide of the formula (II) and water in determined amounts and can find possible applications in cleaners, disinfectant vehicles, laundry detergent, softener and air freshener products. This property dispenses highly precise and costly processes for detergent manufacturers for the production of detergent tablets, toilet blocks, gel detergents and congeners. Also, this property dispenses the usage of a thickener or hardener system, typically a polymeric gelling additive, e.g. polysaccharides, polyethylene glycols, or polyacrylic acid polymers. Moreover, aesthetically, this material aspect allows the detergent producer to develop innovative designs which are attractive to the end consumer and offers different format alternatives.

The inventive compositions are notable for very good storage stability at ambient conditions, i.e. at 40 to 60 % r.h. (relative humidity) and a temperature of 24°C. The inventive compositions in a gel form are soft and easily deformable.

The inventive gel surfactant compositions are preferably transparent.

The inventive compositions possess advantageous adhering properties, in particular to vertical surfaces. Furthermore, the inventive compositions also show advantageous foam-generating properties when brought into contact with water.

The inventive compositions also possess advantageous water solubilities and dissolution rates in water. In sanitary applications such as in a toilet bowl, the inventive compositions in the form of a gel can only be removed completely after numerous flushing processes. For example, 4 g of an inventive composition can preferably only be removed completely after more than 100, more preferably after more than 150 and even more preferably after more than 180 flushes.

Preferably, the inventive compositions comprise 20 to 40 % by weight of the one or more alcohol ethoxylates of the formula (I) of component a), 21 to 29 % by weight of the one or more N-methyl glucamides of the formula (II) of component b) and 33 to 45 % by weight of water of component c), the amounts of the one or more alcohol ethoxylates of the formula (I) of component a), of the one or more N-methyl glucamides of the formula (II) of component b) and of water of component c) being based on the total weight of the composition.

Preferably, R1 in the one or more alcohol ethoxylates of the formula (I) of component a) of the inventive compositions is selected from the group consisting of mixtures of the alkyl group cetyl and the alkenyl group oleyl (i.e. R¹-OH is a mixture of cetyl alcohol and oleyl alcohol).

Generally, alcohol ethoxylates are conversion products of an alcohol with ethylene oxide. The ethoxylation process of the alcohols with ethylene oxide takes place at elevated temperatures and pressures as well as in the presence of a catalyst.

The starting alcohols R¹-OH utilized for the synthesis of the one or more alcohol ethoxylates of the formula (I) of component a) of the inventive compositions, wherein R¹ is a linear or branched, preferably a linear, saturated alkyl group comprising 12 to 22, preferably 12 to 20 and more preferably 16 to 18, carbon atoms or a linear or branched, preferably a linear, mono- or polyunsaturated alkenyl group comprising 12 to 22, preferably 12 to 20 and more preferably 16 to 18, carbon atoms, possess a iodine number of from 30 to 135 g(J₂)/100 g alcohol R¹-OH, preferably of from 36 to 120 g(J₂)/100 g alcohol R¹-OH, even more preferably of from 37 to 105 g(J₂)/100 g alcohol R¹-OH, particularly preferably of from 50 to 95 g(J₂)/100 g alcohol R¹-OH and extraordinarily preferably of from 60 to 75 g(J₂)/100 g alcohol R¹-OH.

Generally, the iodine number, also called iodine adsorption number, is an important characteristic parameter indicating the proportion of unsaturated groups in chemical substances. The iodine number equals the weight of iodine in g required to saturate the unsaturated groups present in 100 g of the respective chemical substance. Chemical substances poor in unsaturated groups expose low iodine numbers, while chemical substances rich in unsaturated groups have high iodine numbers. There are several methods available for the iodine number determination, such as the Kaufmann, Hanus or Wijs method.

The Kaufmann method can be used to determine the iodine number of the alcohol R¹-OH underlying the alcohol ethoxylate of the formula (I). According to this method the alcohol ethoxylate of the formula (I) is mixed with an excess of bromine. This bromine is added to the double bonds in the unsaturated alcohol ethoxylates of the formula (I). This reaction must be carried out in the dark, since the formation of bromine radicals is suppressed by light.

Br2 + R^{a} - CH = CH - R^{b} → R^{a} - CHBr - CHBr - R^{b}

Then the unreacted bromine is reduced to bromide with iodide.

Br₂ + 2I⁻ → I₂ + 2Br⁻

At last, the amount of iodine formed is determined by titration with sodium thiosulfate solution.

I₂ + 2S₂O₃²⁻ → 2I⁻ + S₄O₆²⁻

The result of the titration leads to the iodine number of the alcohol ethoxylates of the formula (I). It is recalculated to relate to the alcohol R¹-OH underlying the alcohol ethoxylate of the formula (I) using the respective molar masses of the alcohol ethoxylate of the formula (I) and the alcohol R¹-OH underlying the alcohol ethoxylate of the formula (I).

In the context of the present invention the iodine numbers given are not related to the alcohol ethoxylates of the formula (I) of the inventive compositions but to the respective alcohol R¹-OH underlying the alcohol ethoxylate and is determined using the Kaufmann method. The iodine numbers given are furthermore related to the total amount of alcohol R¹-OH underlying the one or more alcohol ethoxylates of the formula (I) of component a) of the inventive compositions. This e.g. means that the iodine number given may relate to a mixture of alcohols R¹-OH.

Preferably, the alcohol R¹-OH underlying the respective alcohol ethoxylate of the formula (I) of component a) of the inventive compositions possesses a iodine number of from 37 to 105 g(J₂)/100 g alcohol R¹-OH, more preferably of from 50 to 95 g(J₂)/100 g alcohol R¹-OH and even more preferably of from 60 to 75 g(J₂)/100 g alcohol R¹-OH.

Preferably, in the one or more alcohol ethoxylates of the formula (I) of component a) of the inventive compositions, n, on a molar average, is a number of from 8 to 22.

More preferably, in the one or more alcohol ethoxylates of the formula (I) of component a) of the inventive compositions, n, on a molar average, is a number of from 10 to 20.

Preferably, in the one or more N-methyl glucamides of the formula (II) of component b) of the inventive compositions, RCO is an acyl group comprising linear saturated alkyl groups R with 7 to 17 carbon atoms and linear mono- or polyunsaturated, preferably monounsaturated, alkenyl groups R with 17 carbon atoms.

More preferably, in the one or more N-methyl glucamides of the formula (II) of component b) of the inventive compositions, RCO is a cocoyl group.

In another preferred embodiment of the present invention, R, in the one or more N-methyl glucamides of the formula (II) of component b) of the inventive compositions, is a linear or branched, preferably a linear, saturated alkyl group comprising 11 to 17 and preferably 15 to 17 carbon atoms or a linear or branched, preferably a linear, mono- or polyunsaturated alkenyl group comprising 11 to 17 and preferably 15 to 17 carbon atoms.

The inventive compositions may comprise one or more additional substances in addition to components a), b) and c).

In a preferred embodiment of the invention, the inventive compositions comprise one or more substances selected from the group consisting of other non-ionic surfactants, cationic surfactants, anionic surfactants, amphoteric surfactants, additives and auxiliaries which are customary and specific in each case, for example additives for dissolution time control, additives for performance improvement (e.g. soil release polymers; dye fixatives; dye transfer inhibitors; bleach systems; biocides or antimicrobial components; or builders) or washing assistants (e.g. enzymes; enzyme stabilizers; preservatives; foam enhancers; foam inhibitors; corrosion inhibitors; optical brighteners; UV absorbers; alkalis; hydrotropic compounds; antioxidants; solvents; salts or extenders; dispersants; graying inhibitors; softeners; antistats; dyes; fragrances or perfumes); dyes; pigments; colorants or pearlizing agents.

Other nonionic surfactants that may be contained in the inventive compositions are alkanolamides, alkyldimethyl-amineoxides, di-alkyl-methylamineoxides, alkylamidopropyl-amine oxides, fatty acid-N-methylglucamides different from those of component b) of the inventive compositions, alkylpolyglucosides, oxalkylated fatty acids, alkoxylated alcohols (alkyl ether, alkyl polyethylene glycol ether, fatty alcohol polyglycol ether) different from those of component a) of the inventive compositions, (EO/PO) ethoxylated/propoxylated block copolymers, oxalkylated fatty acid esters and oxalkylated alkylamines. The alkyl and fatty acid groups of these compounds, which also may be fully or partially replaced by the corresponding unsaturated groups, may contain 8 to 22 carbon atoms and may be linear or branched. Oxalkylated means products that contain preferably 1 to 20 units of ethylene oxide or propyleneoxide or mixtures thereof.

The amount of the one or more other nonionic surfactants which can be added to the inventive compositions preferably is of from 0.1 to 50 % by weight and more preferably of from 0.5 to 25 % by weight.

As cationic surfactants there may be used quaternary ammonium compounds such as alkyldimethyl-hydroxyethyl-ammonium. Instead of alkyl these ammonium compounds may also have alkenyl groups or mixtures of both. The alkyl as well as the alkenyl groups may contain 8 to 22 carbon atoms. They may be linear or branched. Preferred ammonium compounds are C₈-C₂₂-alkyl- or alkenyl-dimethyl-hydroxyethyl-ammonium compounds. Particularly preferred ammonium compounds are C₁₂/Cₗ₄-alkyl dimethyl hydroxyethyl ammonium compounds. All mentioned ammonium compounds may contain any kind of anion, the preferred ones are chloride, bromine, acetate, lactate, sulfate or methosulfate. A very preferred ammonium compound is C₁₂/C₁₄-alkyl dimethyl hydroxyethyl ammonium chloride.

Anionic surfactants may be selected from the group consisting of alkyl sulfates, alkyl ether sulfates, alkyl aryl sulfonates such as a linear alkylbenzene sulfonate (LAS), fatty acid soaps, methyl ester sulfonates, paraffin sulfonates, olefin sulfonates, alcohol ethoxysulfates, mono- and dialkyl sulfosuccinates, mono- and dialkyl sulfosuccinamates, sulfotriglycerides, amide soaps, ether carboxylic acids and salts thereof, fatty acid isethionates, fatty acid sarcosinates, fatty acid taurides, N-acyl amino acids such as, for example, acyl lactylates, acyl tartrates, acyl glutamates and alkyl oligoglucoside sulfates.

Furthermore, the compositions according to the invention may contain 0.1 to 12 % by weight and preferably 0.2 to 10 % by weight of amphoteric surfactants. The amphoteric surfactants may be alkyl amidopropyl betaines, alkyl dimethyl betaines, alkyl amphoacetates or -diacetates. The alkyl groups of these compounds, which may be partially or fully replaced by alkenyl groups, may contain 8 to 22 carbon atoms and may be linear or branched. The polyalkylene glycol groups may contain 1 to 20 ethoxy and/or propoxy units.

Depending on the intended use, the formulations according to the invention may comprise, in addition to said surfactants, additives and auxiliaries which are customary and specific in each case, for example additives for dissolution time control or additives for performance improvement.

Suitable additives for dissolution time control are ethanol, isopropanol; butylglycol; di-butylglycol; mono-, di-, tri- or tetra-C₂-C₅ alkylene glycol such as ethylene glycol or propylene glycol, in particular mono-, di- , tri-, or tetra-propylene glycol; polyethylene glycols (PEGs) having molecular weights of at least 400, in particular PEGs of molecular weight ranging from 6,000 to 35,000; glycerol; sugar; and mixtures thereof. In a preferred embodiment of the invention propylene glycol and glycerol are preferred.

Suitable additives for performance improvement include soil release polymers; dye fixatives; dye transfer inhibitors; bleach systems; biocides or antimicrobial components; or builders.

Suitable soil release polymers are copolymers of acrylic acid and maleic acid (Sokalan^{®} CP - BASF), homo- and copolymers of vinylpyrrolidone, vinylimidazole and nonionic monomers (Sokalan^{®} HP - BASF), homopolymers of acrylic acid (Sokalan^{®} PA - BASF), polyethylene terephthalate (PET) and polyoxyethylene terephthalate (POET) (Texcare^{®} products - Clariant). Further suitable soil release polymers are, for example, cellulose ethers or polycondensates based on dibasic carboxylic acids and reactants which possess two or more hydroxyl groups. The dibasic carboxylic acid used is typically terephthalic acid. These soil release polymers may be nonionic or anionic.

The dye fixatives which can be incorporated into inventive compositions are nonionic or cationic and are described below:
Polycondensates which can be used as dye fixatives are obtained by the reaction of cyanamides with aldehydes and ammonium salts and/or monoamines (e.g. dye fixative DF3), by the reaction of monoamines and/or polyamines with epichlorohydrin (e.g. dye fixatives DF2 and DF4) or by the reaction of polyamines with cyanamides and amidosulfuric acid (e.g. dye fixative DF1). The monoamines used may be primary, secondary and tertiary amines. They may be aliphatic amines, for example dialkylamines, especially dimethylamine, alicyclic amines, for example cyclohexylamine, and aromatic amines, for example aniline. However, the amines used may also simultaneously have aliphatic, alicyclic and aromatic substituents. In addition, it is also possible to use heterocyclic compounds, for example pyridine. The term "polyamines" here includes, for example diamines, triamines, tetraamines, etc, and also the analogous N-alkylpolyamines and N,N-dialkylpolyamines. Examples thereof are ethylenediamine, propylenediamine, butylenediamine, pentylenediamine, hexylenediamine, diethylenetriamine, triethylenetetraamine and higher polyamines. Particularly preferred polyamines are ethylenediamine, diethylenetriamine and dimethylaminopropylamine. The ammonium salts are salts of ammonia, especially ammonium chloride or the abovementioned amines or polyamines with different inorganic or organic acids, or else quaternary ammonium salts.
   The cyanamides may be cyanamide or dicyandiamide.

Aldehydes which can be used for the synthesis of the dye fixatives are, for example, aliphatic aldehydes, for example formaldehyde, acetaldehyde, propionaldehyde, butyraldehyde; dialdehydes, for example glyoxal; unsaturated aldehydes, for example acrolein, crotonaldehyde and aromatic aldehydes, for example benzaldehyde. Particular preference is given to the aliphatic aldehydes, especially formaldehyde.
The dye fixatives used may also be homo- and copolymers based on diallyldimethylammonium chloride (DADMAC) (e.g. dye fixatives DF5, DF6 and DF7). Copolymers based on DADMAC contain, as further components, other vinylic monomers, for example vinylimidazole, vinylpyrrolidone, vinyl alcohol, vinyl acetate, (meth)acrylic acid/ester, acrylamide, styrene, styrenesulfonic acid, acrylamidomethylpropanesulfonic acid, etc. Homopolymers based on DADMAC are obtainable under the trade names Dodigen^{®} 3954, Dodigen^{®} 4033 and Genamin PDAC (from Clariant).

Bleach systems such as inorganic peroxygen compounds, especially hydrogen peroxide and solid peroxygen compounds which dissolve releasing hydrogen peroxide in water, such as sodium perborate and sodium carbonate perhydrate and mixtures thereof; and bleach activators, such as those from the substance classes of the N- or O-acyl compounds, for example polyacylated alkylenediamines, especially tetraacetylethylene-diamine and tetraacetylglycoluril, N-acylated hydantoins, hydrazides, triazoles, hydrotriazines, urazoles, diketopiperazines, sulfurylamides and cyanurates, and also carboxylic anhydrides, especially phthalic anhydride and substituted maleic anhydrides, carboxylic esters, especially sodium acetoxybenzenesulfonate, sodium benzoyloxybenzenesulfonate (BOBS), sodium nonanoyloxybenzenesulfonate (NOBS), sodium isononanoyloxy-benzenesulfonate (ISONOBS), and acylated sugar derivatives such as pentaacetylglucose, and mixtures thereof.

Biocides or antimicrobial components are any known ingredient having the ability of reducing or eliminating by killing or removing the micro-organisms existing on a surface. Biocides or antimicrobial components useful herein include alcohols, aldehydes, formaldehyde releasing compounds, phenolics, acid esters carbamates, amides, dibenzamidines, pyridine derivatives and related compounds, azoles, heterocyclic N,S compounds, N haloalkylthio compounds, compounds with activated halogen atoms, surface active agents, organometallic compounds, thiocyanates, biphenyl, triazine, oxidizing agents and mixtures thereof.

Suitable builders are organic and inorganic builders and are neutral or, in particular, alkaline salts which are able to precipitate out calcium ions or bind calcium ions to form a complex. Suitable and particularly ecologically acceptable builder substances, such as finely crystalline, synthetic hydrous zeolites preferably the type NaA, which have a calcium-binding capacity in the range from 100 to 200 mg of CaO/g, are used in preference. Zeolite and phyllosilicates can be present in the composition in an amount up to 20 % by weight. Organic builders which can be used are, for example, the group consisting of amino carboxylic acid, organo aminophosphonic acid compounds, and mixtures thereof. Those components, which are acidic in nature, having for example phosphonic acid or carboxylic acid functionalities, may be present either in their acid form or as a complex/salt with a suitable counter cation such as an alkali or alkaline metal ion, ammonium, or substituted ammonium ion, or any mixtures thereof. Suitable components for use herein include the amino carboxylic acids such as ethylenediamine-N,N'-disuccinic acid (EDDS), ethylenediamine tetraacetic acid (EDTA), N-hydroxyethylenediamine triacetic acid, nitrilotriacetic acid (NTA), ethylene diamine tetrapropionic acid, ethylenediamine-N,N'-diglutamic acid, 2-hydroxypropylenediamine-N,N'-disuccinic acid, triethylenetetraamine hexacetic acid, diethylenetriamine pentaacetic acid (DETPA), trans 1,2 diaminocyclohexane-N,N,N',N'-tetraacetic acid or ethanoldiglycine. Other suitable components for use herein include the organo aminophosphonic acids such as ethylenediamine tetrakis (methylenephosphonic acid), diethylene triamine-N,N,N',N",N"-pentakis (methylene phosphonic acid) (DETMP), 1-hydroxyethane 1,1-diphosphonic acid (HEDP) or hydroxyethane dimethylenephosphonic acid. It is also possible to use polymeric carboxylates and salts thereof. These include, for example, the salts of homopolymeric or copolymeric polyacrylates, polymethylacrylates and in particular, copolymers of acrylic acid with maleic acid, and also polyvinylpyrrolidone and urethanes. The relative molecular mass of the homopolymers is generally between 1000 and 100,000, that of the copolymers is between 2000 and 200,000, preferably 50,000 to 120,000, based on the free acid, in particular water-soluble polyacrylates which have been crosslinked, for example, with approximately 1 % of a sugar polyallyl ether and which have a relative molecular mass above one million are also suitable. Examples thereof are the polymers obtainable under the name Carbopol^{®} 940 and 941.

In general, additionally present in small use concentrations are additive constituents which can be summarized under the term "washing assistants" and which thus include different active substance groups such as for example enzymes, especially proteases, lipases, cellulases, amylases and mannanases; enzyme stabilizers; preservatives; foam enhancers; foam inhibitors such as silicone oils or paraffins; corrosion inhibitors; optical brighteners; UV absorbers; alkalis; hydrotropic compounds; antioxidants; solvents; salts or extenders; dispersants; graying inhibitors; softeners; antistats; dyes, fragrances and perfumes.

Suitable enzymes are those from the class of proteases, lipases, amylases and their mixture. Their proportion can be from 0.1 to 1 % by weight. The enzymes can be adsorbed to carrier substances and/or embedded into coating substances.

Suitable preservatives are, for example, phenoxy ethanol, formaldehyde solution, pentanediol or sorbic acid.

In a further preferred embodiment of the invention the inventive compositions additionally contain one or more solvents, preferably lower alkyl ethers of ethylene glycol, propylene glycol, polyethylene glycol and polypropylene glycol. "Lower alkyl" preferably means alkyl groups with 1 to 4 carbon atoms.

Suitable salts or extenders are, for example, sodium chloride, sodium sulfate, sodium carbonate, sodium silicate (water glass), magnesium chloride, or magnesium sulfate.

The aesthetics of the inventive compositions may be further enhanced by the addition of a dye, pigment, colorant or a pearlizing agent. Typical examples of dyes are methylene blue (Basic Blue 9), Direct Yellow 50, Solvent Red 17, Reactive Green 12 (Phthalocyanine, Clariant).

In a more preferred embodiment of the present invention, the inventive compositions comprise one or more substances selected from the group consisting of preservatives, antimicrobial components, enzymes, dye transfer inhibitors, soil release polymers, cationic surfactants, anionic surfactants, amphoteric surfactants, propylene glycol, glycerol, dyes, pigments and fragrances.

In an even more preferred embodiment of the present invention, the inventive compositions comprise one or more substances selected from the group consisting of propylene glycol and glycerol.

Preferably, the inventive compositions are solid at a temperature of 20 °C.

More preferably, the inventive compositions are present, at a temperature of 20 °C, in the form of a gel or in the form of a paste and preferably in the form of a gel. Among the inventive compositions being present, at a temperature of 20 °C, in the form of a gel, inventive compositions in monodose format or in the form of a monodose product constitute a preferred embodiment of the invention.

Even more preferably, the viscosity of the inventive compositions, measured at a temperature of 20 °C and a shear stress of 1000 Pa, is of from 10 to 100000 Pa · s and preferably of from 10 to 25000 Pa · s.

In a particularly preferred embodiment of the invention the inventive compositions are present in the form of a paste and the viscosity of these inventive compositions, measured at a temperature of 20°C and a shear stress of 1000 Pa, is of from 10 to 200 Pa · s.

In another particularly preferred embodiment of the invention the inventive compositions are present in the form of a gel and the viscosity of these inventive compositions, measured at a temperature of 20°C and a shear stress of 1000 Pa, is of from 300 to 100000 Pa · s and preferably of from 300 to 25000 Pa · s.

The viscosities of the inventive compositions are measured using a rheometer Haake Mars III (Thermofisher Scientific) with a 35 mm plate-plate geometry at 20 °C.

Especially preferred inventive compositions exhibit viscoelastic properties and no flow when in the steady-state.

In a preferred embodiment of the present invention, the inventive compositions are present in the form of a monodose product and more preferably in the form of a monodose product and, at a temperature of 20 °C, in the form of a gel.

Preferably, the average dissolution rate of the inventive compositions in water at a temperature of from 20 to 40 °C is of from 1 to 100 mg/minute and preferably of from 10 to 80 mg/minute.

Furthermore preferably, the average dissolution rate of the inventive compositions in water at a temperature of 20 °C is of from 1 to 40 mg/minute and preferably of from 10 to 40 mg/minute and at a temperature of from 30 to 40 °C is of from 40 to 100 mg/minute and preferably of from 40 to 80 mg/minute.

Preferably, the inventive compositions are adhering to vertical surfaces and more preferably to vertical surfaces made of ceramics, plastic or stainless steel.

Preferably, the inventive compositions generate foam when rinsed or flushed with water.

The inventive compositions, preferably being present, at a temperature of 20 °C, in the form of a gel or in the form of a paste and more preferably in the form of a gel, are preferably obtainable by heating the components a), b) and c) of the inventive compositions at a temperature of from 45 to 80 °C and more preferably of from 50 to 75 °C, preferably under mixing, until a homogeneous melt is obtained and then cooling the homogeneous melt to a temperature of from 20 to 40 °C.

In a preferred embodiment of the present invention the inventive compositions, preferably being present, at a temperature of 20 °C, in the form of a gel or in the form of a paste and more preferably in the form of a gel, comprise one or more additional substances in addition to components a), b) and c) and are obtainable by adding the one or more additional substances either before or during the heating of components a), b) and c) or during the cooling of the homogeneous melt.

Inventive compositions consisting of components a), b) and c) generally are present in the form of gels. They may also be present in the form of gels if they comprise additional substances in addition to components a), b) and c) in moderate amounts, such as propylene glycol and glycerol in amounts of 10 % by weight or less and preferably in amounts of 8 % by weight or less. The inventive compositions may be present in the form of a paste if they comprise additional substances in addition to components a), b) and c) in relatively high amounts. Pastes can also be obtained in case anionic surfactants are present in the inventive compositions, also in moderate amounts, for example anionic surfactants such as sodium lauryl ether sulfate comprising 2 ethylene oxide units in an amount of approximately 10 % by weight.

In a further preferred embodiment of the present invention the inventive compositions consist of the components a), b) and c). In another preferred embodiment of the present invention the inventive compositions comprise components a), b) and c) and optionally one or more additional substances in addition to components a), b) and c), these one or more additional substances, if present, being contained in the inventive compositions preferably in an amount of 40 % by weight or less, more preferably in an amount of 10 % by weight or less and even more preferably in an amount of 8 % by weight or less, the amounts of the one or more additional substances being based on the total weight of the composition. Among the inventive compositions comprising one or more additional substances those inventive compositions comprising one or more substances selected from the group consisting of propylene glycol and glycerol are preferred. In a particularly preferred embodiment of the invention the inventive compositions consist of components a), b) and c) and one or more additional substances selected from the group consisting of propylene glycol and glycerol.

The inventive compositions are advantageously suited for cleaning, scenting or disinfection purposes, preferably in home or personal care applications.

Therefore, a further subject matter of the present invention is the use of the inventive compositions for cleaning, scenting or disinfection purposes, preferably in home or personal care applications.

Due to the presence of the one or more alcohol ethoxylates of the formula (I) of component a) and the one or more N-methyl glucamides of the formula (II) of component b), the inventive compositions consisting of components a), b) and c), i.e. without the presence of further substances in addition to components a), b) and c), could be used for cleaning purposes or cleaning applications. However, also for this purpose or application the addition of further substances in addition to components a), b) and c) to the inventive compositions is possible. For scenting or disinfection purposes or applications components a), b) and c) of the inventive compositions may e.g. be used as a base composition or a "chassis", i.e. for these purposes or applications the inventive compositions might comprise additional substances in addition to components a), b) and c), such as components with respective activities, e.g. perfumes or components with disinfection properties.

During the inventive use the inventive compositions are preferably applied to surfaces made of ceramics, plastic or stainless steel, more preferably to toilet ceramics, or are applied in laundry machines or dishwashers.

The inventive compositions present in the form of a paste may preferably be used in hand dishwashing, laundry and hard surface cleaning applications.

The inventive compositions present in the form of a gel may preferably be used in laundry, automatic dishwashing, hand dishwashing, hard surface cleaning, toilet, air care and personal care applications.

As already stated, the inventive compositions, preferably being present, at a temperature of 20 °C, in the form of a gel or in the form of a paste and more preferably in the form of a gel, may be prepared by heating the components a), b) and c) of the inventive compositions at a temperature of from 45 to 80 °C and preferably of from 50 to 75 °C, preferably under mixing, until a homogeneous melt is obtained and then cooling the homogeneous melt to a temperature of from 20 to 40 °C. The inventive compositions may comprise one or more additional substances in addition to the components a), b) and c). In the process for the preparation of the inventive compositions these one or more additional substances in addition to components a), b) and c) may either be added before or during the heating of the components a), b) and c) or during the cooling of the homogeneous melt.

Therefore, a further subject matter of the invention is a process for the preparation of inventive compositions, preferably being present, at a temperature of 20°C, in the form of a gel or in the form of a paste and more preferably in the form of a gel, whereby the components a), b) and c) of the inventive compositions are heated at a temperature of from 45 to 80 °C and preferably of from 50 to 75 °C, preferably under mixing, until a homogeneous melt is obtained and then the homogeneous melt is cooled to a temperature of from 20 to 40 °C.

In a preferred embodiment of the inventive process, inventive compositions comprising one or more additional substances in addition to components a), b) and c) and preferably being present, at a temperature of 20°C, in the form of a gel or in the form of a paste and more preferably in the form of a gel, are prepared, and, in the inventive process, the one or more additional substances in addition to components a), b) and c) are either added before or during the heating of components a), b) and c) or during the cooling of the homogeneous melt.

The examples below serve to illustrate the invention in more detail without limiting it thereto. All amounts are given as % by weight (wt.-%).

In the examples the following substances have been used:
- Dodigen^{®} 226: Cocos alkyl dimethyl benzyl ammonium chloride (Clariant, 50 wt.-% in water)
- Genapol^{®} O 100: Cetyl oleyl alcohol ethoxylate with 10 ethylene oxide (EO) units (Clariant, 100 wt.-%; iodine number related to alcohol underlying Genapol^{®} O 100: 60 to 75 g(J₂)/100 g alcohol)
- Genapol^{®} O 200: Cetyl oleyl alcohol ethoxylate with 20 ethylene oxide (EO) units (Clariant, 100 wt.-%; iodine number related to alcohol underlying Genapol^{®} O 200: 60 to 75 g(J₂)/100 g alcohol)
- Genapol^{®} T 250: Tallow alkyl ethoxylate with 25 ethylene oxide (EO) units (Clariant, 100 wt.-%; iodine number related to alcohol underlying Genapol^{®} T 250: < 1 g(J₂)/100 g alcohol)
- Glucopure^{®} Foam: Cocoyl N-methyl glucamide (Clariant, 40 wt.-% in water and small amounts of propylene glycol and glycerol)
- Glucopure^{®} Wet: N-C₈/C₁₀ acyl N-methyl glucamide (Clariant, 50 wt.-% in water and small amounts of propylene glycol)

"Cetyl oleyl alcohol" designates a mixture of cetyl alcohol and oleyl alcohol.

The fatty acids R-COOH underlying the cocoyl N-methyl glucamide of Glucopure^{®} Foam are linear C₈-C₁₈ fatty acids. In approximately 86 wt.-% of the fatty acids R-COOH underlying the cocoyl N-methyl glucamide of Glucopure^{®} Foam the residue R is selected from the group consisting of linear saturated alkyl groups with 11 to 17 carbon atoms and linear monounsaturated alkenyl groups with 17 carbon atoms.

In the following Table 1, "na" means "not applicable".

**Table 1: Examples 1 - 6**

| Component | Weight-% of active component | | | | | |
|---|---|---|---|---|---|---|
| | Example 1 (inventive) | Example 2 (inventive) | Example 3 (comparative) | Example 4 (inventive) | Example 5 (comparative) | Example 6 (inventive) |
| Cocoyl N-methyl glucamide | 25.2 | 28.8 | 0 | 25.2 | 25.2 | 24.7 |
| N-C₈/C₁₀ acyl N-methyl glucamide | 0 | 0 | 32.2 | 0 | 0 | 0 |
| Cetyl oleyl alcohol ethoxylate with 10 ethylene oxide (EO) units | 30.0 | 20.0 | 30.0 | 0 | 0 | 30.0 |
| Cetyl oleyl alcohol ethoxylate with 20 ethylene oxide (EO) units | 0 | 0 | 0 | 30.0 | 0 | 0 |
| Tallow alkyl ethoxylate with 25 ethylene oxide (EO) units | 0 | 0 | 0 | 0 | 30.0 | 0 |
| Cocos alkyl dimethyl benzyl ammonium chloride | 0 | 0 | 0 | 0 | 0 | 0.8 |
| Water | 39.2 | 44.8 | 34.3 | 39.2 | 39.2 | 40.2 |
| Propylene glycol and Glycerol | rest | Rest | Rest (Glycerol = 0) | rest | rest | rest |
| Physical state of the sample after cooling down to 25 °C | Gel | Gel | Liquid phase, no gel formation | Gel | Inhomogeneous phase, no gel formation | Gel |
| Stability under storage at 40 - 60 % r.h., 24 °C | stable gel phase > 3 months | stable gel phase > 3 months | Na | stable gel phase > 3 months | na | stable gel phase > 3 months |

The compositions of the inventive Examples 1, 2, 4 and 6 and of the Comparative Examples 3 and 5 have been prepared in the following way: components a), b) and c) were mixed and heated to 55 to 75 °C until a homogeneous melt was obtained. Afterwards the homogeneous melt was cooled down to 25 °C.

All gels of Examples 1, 2, 4 and 6 are soft and deformable. They may easily be transformed into monodose formats or monodose products by either casting the homogeneous melt of the preparation process into a respective form or by cutting and/or pressing the gels in order to obtain the desired form.

The gel of Example 1 was further examined:
The gel of Example 1 shows a yield stress at 181 Pa. Furthermore, the viscosity at a shear stress of 1000 Pa is 16000 Pa · s. Viscosity characterization was done using a rheometer Haake Mars III (Thermofisher Scientific) with a 35 mm plate-plate geometry at 20 °C.

The average dissolution rate of the gel of Example 1 in water at a temperature of 20 °C is 33 mg/minute and at a temperature between 30 °C and 40 °C is of from 40 to 60 mg/minute.

The gel of Example 1 was applied as a monodose product to the ceramics of a toilet bowl and the adhesive properties of the gel have been tested. The gel could only be removed completely after approximately 180 flushing operations which demonstrates that the gel of Example 1 possesses very advantageous adhesive properties.

The inventive gel of Example 6 comprises 0.8 wt.-% of cocos alkyl dimethyl benzyl ammonium chloride which has disinfectant properties and therefore, the gel of Example 6 could be used for disinfection purposes or in disinfection applications.

## Claims

1. A composition comprising
a) 5 to 50 % by weight and preferably 20 to 50 % by weight of one or more alcohol ethoxylates of the formula (I)
R¹-O-(CH₂CH₂O)ₙ-H (I)
wherein
R¹ is a linear or branched, preferably a linear, saturated alkyl group comprising 12 to 22, preferably 12 to 20 and more preferably 16 to 18, carbon atoms or a linear or branched, preferably a linear, mono-or polyunsaturated alkenyl group comprising 12 to 22, preferably 12 to 20 and more preferably 16 to 18, carbon atoms,
whereby the alcohol R¹-OH underlying the respective alcohol ethoxylate of the formula (I) possesses a iodine number of from 30 to 135 g(J₂)/100 g alcohol R¹-OH and preferably of from 36 to 120 g(J₂)/100g alcohol R¹-OH, and
n on a molar average, is a number of from 4 to 50 and preferably of from 5 to 25, and
b) 15 to 50 % by weight and preferably 17 to 44 % by weight of one or more N-methyl glucamides of the formula (II) 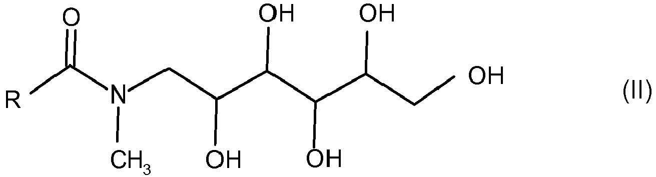 wherein
R is a linear or branched, preferably a linear, saturated alkyl group comprising 7 to 21 and preferably 7 to 17 carbon atoms or a linear or branched, preferably a linear, mono- or polyunsaturated alkenyl group comprising 7 to 21 and preferably 7 to 17 carbon atoms,
whereby in at least 25.0 wt.-%, preferably in at least 50.0 wt.-%, more preferably in at least 65.0 wt.-% and even more preferably in at least 80.0 wt.-% of the fatty acids R-COOH underlying the respective N-methyl glucamides of the formula (II) R is a linear or branched, preferably a linear, saturated alkyl group comprising 11 to 21 and preferably 11 to 17 carbon atoms or a linear or branched, preferably a linear, mono- or polyunsaturated alkenyl group comprising 11 to 21 and preferably 11 to 17 carbon atoms, and
c) 25 to 45 % by weight and preferably 30 to 45 % by weight of water,
the amounts of the one or more alcohol ethoxylates of the formula (I) of component a), of the one or more N-methyl glucamides of the formula (II) of component b) and of water of component c) being based on the total weight of the composition.

2. Composition according to claim 1, **characterized in that** it comprises 20 to 40 % by weight of the one or more alcohol ethoxylates of the formula (I) of component a), 21 to 29 % by weight of the one or more N-methyl glucamides of the formula (II) of component b) and 33 to 45 % by weight of water of component c), the amounts of the one or more alcohol ethoxylates of the formula (I) of component a), of the one or more N-methyl glucamides of the formula (II) of component b) and of water of component c) being based on the total weight of the composition.

3. Composition according to claim 1 or 2, **characterized in that** R¹ in the one or more alcohol ethoxylates of the formula (I) of component a) is selected from the group consisting of mixtures of the alkyl group cetyl and the alkenyl group oleyl.

4. Composition according to one or more of claims 1 to 3, **characterized in that** the alcohol R¹-OH underlying the respective alcohol ethoxylate of the formula (I) of component a) possesses a iodine number of from 37 to 105 g(J₂)/100 g alcohol R¹-OH, preferably of from 50 to 95 g(J₂)/100 g alcohol R¹-OH and more preferably of from 60 to 75 g(J₂)/100 g alcohol R¹-OH.

5. Composition according to one or more of claims 1 to 4, **characterized in that** in the one or more alcohol ethoxylates of the formula (I) of component a), n, on a molar average, is a number of from 8 to 22.

6. Composition according to claim 5, **characterized in that** in the one or more alcohol ethoxylates of the formula (I) of component a), n, on a molar average is a number of from 10 to 20.

7. Composition according to one or more of claims 1 to 6, **characterized in that** in the one or more N-methyl glucamides of the formula (II) of component b), RCO is an acyl group comprising linear saturated alkyl groups R with 7 to 17 carbon atoms and linear mono- or polyunsaturated, preferably monounsaturated, alkenyl groups R with 17 carbon atoms.

8. Composition according to one or more of claims 1 to 7, **characterized in that** in the one or more N-methyl glucamides of the formula (II) of component b), RCO is a cocoyl group.

9. Composition according to one or more of claims 1 to 8, **characterized in that** it comprises one or more substances selected from the group consisting of preservatives, antimicrobial components, enzymes, dye transfer inhibitors, soil release polymers, cationic surfactants, anionic surfactants, amphoteric surfactants, propylene glycol, glycerol, dyes, pigments and fragrances.

10. Composition according to one or more of claims 1 to 9, **characterized in that** it comprises one or more substances selected from the group consisting of propylene glycol and glycerol.

11. Composition according to one or more of claims 1 to 10, **characterized in that** it is solid at a temperature of 20 °C.

12. Composition according to one or more of claims 1 to 11, **characterized in that** it is present, at a temperature of 20 °C, in the form of a gel or in the form of a paste and preferably in the form of a gel.

13. Composition according to one or more of claims 1 to 12, **characterized in that** its viscosity, measured at a temperature of 20 °C and a shear stress of 1000 Pa, is of from 10 to 100000 Pa · s and preferably of from 10 to 25000 Pa · s.

14. Composition according to one or more of claims 1 to 13, **characterized in that** it is present in the form of a monodose product and preferably in the form of a monodose product and, at a temperature of 20 °C, in the form of a gel.

15. Composition according to one or more of claims 1 to 14, **characterized in that** its average dissolution rate in water at a temperature of from 20 to 40 °C is of from 1 to 100 mg/minute and preferably of from 10 to 80 mg/minute.

16. Composition according to one or more of claims 1 to 15, **characterized in that** it is adhering to vertical surfaces and preferably to vertical surfaces made of ceramics, plastic or stainless steel.

17. Composition according to one or more of claims 1 to 16, **characterized in that** it generates foam when rinsed or flushed with water.

18. Composition according to one or more of claims 1 to 17, preferably being present, at a temperature of 20 °C, in the form of a gel or in the form of a paste and more preferably in the form of a gel, **characterized in that** it is obtainable by heating the components a), b) and c) of the composition at a temperature of from 45 to 80 °C and preferably of from 50 to 75°C, preferably under mixing, until a homogeneous melt is obtained and then cooling the homogeneous melt to a temperature of from 20 to 40 °C.

19. Composition according to claim 18, preferably being present, at a temperature of 20 °C, in the form of a gel or in the form of a paste and more preferably in the form of a gel, **characterized in that** it comprises one or more additional substances in addition to components a), b) and c) and is obtainable by adding the one or more additional substances either before or during the heating of components a), b) and c) or during the cooling of the homogeneous melt.

20. Use of a composition according to one or more of claims 1 to 19 for cleaning, scenting or disinfection purposes, preferably in home or personal care applications.

21. Process for the preparation of a composition according to one or more of claims 1 to 19, preferably being present, at a temperature of 20 °C, in the form of a gel or in the form of a paste and more preferably in the form of a gel, **characterized in that** components a), b) and c) of the composition are heated at a temperature of from 45 to 80 °C and preferably of from 50 to 75°C, preferably under mixing, until a homogeneous melt is obtained and then the homogeneous melt is cooled to a temperature of from 20 to 40 °C.

22. Process according to claim 21, **characterized in that** a composition according to one or more of claims 1 to 19 comprising one or more additional substances in addition to components a), b) and c) and preferably being present, at a temperature of 20 °C, in the form of a gel or in the form of a paste and more preferably in the form of a gel, is prepared, and that, in the process, the one or more additional substances in addition to components a), b) and c) are either added before or during the heating of components a), b) and c) or during the cooling of the homogeneous melt.
